# EUROPEAN PATENT SPECIFICATION

(11) **EP 3 863 691 B1**
(45) Date of publication and mention of the grant of the patent: **20.12.2023**
(21) Application number: 19828878.9
(22) Date of filing: 11.10.2019
(51) Int. Cl.: A61L 27/12, A61L 27/36, A61L 27/54

(54) **BIOACTIVE BONE REPAIR PARTICLES**
BIOAKTIVE KNOCHENREPARATURPARTIKEL
PARTICULES BIOACTIVES DE RÉPARATION OSSEUSE

(30) Priority: 11.10.2018 NL 2021794
(43) Date of publication of application: 18.08.2021
(73) Proprietor: Access2Bone IP B.V., 2333 BD Leiden (NL)
(72) Inventor: VAN DE MORTEL, Nol, 2333 BD Leiden (NL); SICKLER, Michael, 2333 BD Leiden (NL); DE ROODE-BEULING, Nienke, 2333 BD Leiden (NL)
(74) Representative: Nederlandsch Octrooibureau
(86) International application number: PCT/NL2019/050675
(87) International publication number: WO 2020/076158

(56) References cited:
- US-A1- 2016 184 390

## Description

### Field of the invention

The present invention relates to a method for preparing bioactive bone repair substrate comprising granules, to the bioactive bone repair substrate comprising granules and to the application of such substrates in the field of regenerative medicine, in particular in bone and connective tissue engineering.

### Background art

Every year, more than two million bone graft procedures are performed globally to address bone fractures and other orthopaedic-related injuries resulting from a variety of surgical, degenerative and traumatic causes. The repair of bone defects can be facilitated by placing a bone repair material in the defect site, where a loss of natural bone has occurred or where bone repair is impaired. The bone repair material is meant to selectively and over a sustained period of time promote the regeneration of natural bone structures.

The current clinical gold standard of treatment is autologous bone, harvested primarily from the patient's iliac crest or other locations such as the distal femur, proximal tibia, ribs and intramedullary canal. Autologous bone provides an osteoconductive three-dimensional scaffold for bone growth, osteogenic cells and osteoinductive growth factors. Despite its immunocompatibility and excellent osteoconductive as well as osteoinductive properties, autograft bone is of limited supply and is often associated with limitations such as the need for additional surgery, pain at the donor site, morbidity and a high and unpredictable resorption. Moreover, this procedure is not suitable for elderly people and people with impaired bone metabolism for example in subjects suffering from osteoporosis, diabetes mellitus and obesitas.

These limitations have led to a continued search for alternatives. Synthetic bone repair materials typically comprise calcium phosphate based products or materials. In contrast to naturally-occurring bone repair materials, synthetic bone repair materials are often less osteoconductive and hardly osteoinductive. Much research has been, and still is, directed towards improved synthetic bone repair materials. The development of numerous alternative materials for filling bone defects, such as allografts, xenografts, and synthetic materials have resulted from a better understanding of the biology of the healing of bones and technological development. Most of these graft materials used clinically are osteoconductive, which enhances the migration of osteogenic cells. The capacity of the graft materials to induce osteogenesis is called osteoinductivity. The foreign body reactions of graft material may significantly hinder the regeneration of bone and the osseointegration of material used for filling bone defects though. Therefore the therapeutic effects of alternative graft materials for filling bone defects for bone repair are far from satisfactory.

Bone repair and regeneration is a complex cascade of biological events, which involves not only extracellular matrix components and bone-forming cells, but also osteoinductive and osteogenic growth factors. A major challenge in the field is finding an optimal carrier for protein delivery at the site of injury. Even though diverse growth factor delivery systems have been intensively investigated in orthopaedics, there is still need to improve delivery of bioactive osteogenic proteins capable of promoting bone repair or regeneration from a bone repair material.

The application of osteogenic growth factors such as bone morphogenic protein 2 (BMP-2) or transforming growth factor beta (TGF-β) by adsorbing them superficially onto a biomimetic scaffold only gives a short-term burst release of BMP2 release which is associated with undesired side effects and the osteoinductive effects of these agents are restricted both temporally and spatially. Carrier coatings that can release the growth factor BMP-2 sequentially have been developed for a more sustained release, such as those described in WO2006/016807. A calcium phosphate (CaP) coating with BMP2 incorporated indeed enhanced osteoinductivity yet still suffers from the drawback that the growth factor is not homogeneously distributed throughout the whole substrate; steady release for only up to 5 weeks has been reported; the coating process is labour-intensive and expensive. The process involves co-precipitation of said bioactive material with the other coating components and is labour intensive, difficult to scale up and is accompanied with a significant loss of valuable bioactive material during the coating process due to adhesion to the reaction vessels. In US2016/0184390 a graft matrix providing for sustained BMP-2 release has been developed wherein calcium granules with both micro-and macropores into which the osteoinductive protein is absorbed are described.

### Summary of the invention

The present inventors have overcome these disadvantages in the prior art and developed a method for preparing bioactive bone repair substrate comprising granules with a homogeneous distribution of bioactive materials in a calcium phosphate matrix. According to the present invention a bioactive material is incorporated into a calcium phosphate substrate of which bioactive bone repair granules are formed through setting and granulation. The present method avoids the use of tedious methods of depositing a coating of calcium phosphate layers on a substrate such as there are described in Proc. Instr. Mech. Engrs vol 212 part H by K. de Groot *et al.* It is also an object of the present invention to provide a process for incorporating a bioactive material into a bone repair substrate which is carried out batchwise. This is a simplified and controlled process limiting loss of valuable materials.

It is a further object of the invention to provide bioactive bone repair substrate comprising granules with bioactive material derived from bone, preferably from demineralized bone matrix, wherein advantageously low amounts of bioactive material are used. About 100 micrograms of bioactive material derived from bone is used per gram of end product. Without wishing to be bound to any theory, the bioactive bone repair substrate with homogeneously distributed bioactive material therein allows for constant and sustained release and biological availability of bioactive material over time; it thus allows for a continuous signalling function of the different osteoconductive and osteoinductive factors obtained from demineralized bone matrix. This is thought to support bone repair *in situ* by mimicking or enhancing the growth factor signalling involving several different factors in physiological bone repair. This is clearly beneficial compared to the injection of bioactive material extracted from demineralized bone matrix alone into, onto, or near bone defect sites in the prior art; in the prior art, the effectiveness of the bioactive material is hampered by the high availability of all the osteoinductive and osteoconductive factors all at once, thus lacking a continuous signalling function. It is further believed that where normally demineralized bone matrix of a single donor provides bioactive material for up to about one hundred patients or treatment sides, the use of bioactive material extracted from demineralized bone matrix of a single donor in the bioactive bone repair substrate according to the invention provides bioactive bone repair substrate for 1000 to 10.000 patients or treatment sides. As donor bone is of limited supply, the invention advantageously increases the treatment potential of bioactive material obtained from demineralized bone matrix.

When granules are prepared from a set calcium phosphate matrix with a homogeneous distribution of bioactive material therein, a constant and better controlled release of bioactive material is achieved, over prolonged time periods, i.e. sustained release. The desire in the art to prolong growth factor or osteogenic protein retention highlights the importance of maintaining protein bioactivity over extended periods of time *in situ.*

It is a further object of the invention to provide for a bone repair granulate substrate wherein a bioactive material is incorporated which is particularly useful in connective tissue and bone tissue engineering or regeneration of bone fractures.

These and other objectives are set out in the detailed description of the invention.

### List of figures

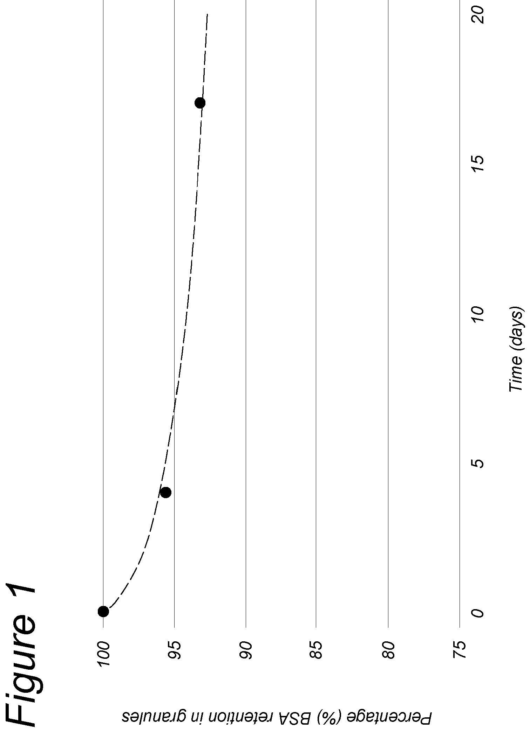

Figure 1 shows the *in vitro* loading and retention profiles for BSA incorporated in a calcium deficient hydroxyapatite granule over a period of 17 days. The percentage (%) of retention of BSA incorparated in the granules on the Y-axis is plotted against time (in days) on the x-axis, whereby the dashed line fitted through the measurement points serves as a guide to the eye.

### Description of embodiments

The invention relates to a bioactive bone repair substrate comprising granules obtainable by or obtained from a set solid state mixture of a calcium phosphate based matrix and a bioactive material, and/or a bioactive bone repair substrate comprising granules from a calcium phosphate based matrix and at least one bioactive material dispersed in said matrix, said substrate exhibiting sustained release of the at least one bioactive material, preferably exhibiting release of the at least one bioactive material.

The invention also relates to a bioactive bone repair substrate comprising granules from a calcium phosphate based matrix and at least one bioactive material obtainable by or obtained from a) admixing a calcium phosphate powder and a bioactive material extracted from bone, b) allowing the mixture to set to a solid state, c) forming granules from the set solid state, preferably to granules having an average diameter of 25 - 10,000 µm.

In the method according to the invention bioactive bone repair granules are prepared such that, when applied *in vivo* allow for a slow or gradual or sustained or extended release of the bioactive material. In a preferred embodiment the sustained release of bioactive material is achieved for the complete time of *in vivo* turnover of the osteoconductive matrix or the *in vivo* replacement of calcium phosphate with bone. The sustained release of bioactive material can be characterized by a controlled and steady release over at least that period. In an embodiment the release of bioactive material continues preferably for at least 4 weeks, more preferably up to 8 weeks, more preferably up to 3 months, even more preferably up to 6 months after start of treatment.

In one aspect a method is provided for preparing a bioactive bone repair substrate comprising granules from a calcium phosphate based matrix and at least one bioactive material, said method comprising: (a) providing a calcium phosphate powder, (b) providing an aqueous solution comprising a bioactive material; (c) admixing components (a) and (b), to obtain a mixture, and (d) allowing the mixture to set to a solid state, (e) forming granules from the set solid state, and (f) selecting the granules based on size, preferably granules having an average diameter of 25 - 10,000 µm.

In one aspect a method is provided for preparing a bioactive bone repair substrate comprising granules from a set solid state mixture of a calcium phosphate based matrix and bioactive material extracted from bone, preferably from demineralized bone matrix, said method comprising: (a) providing a calcium phosphate powder, (b) providing an aqueous solution comprising bioactive material derived from bone, preferably bioactive material derived from human demineralized bone matrix; (c) admixing components (a) and (b), to obtain a mixture, and (d) allowing the mixture to set to a solid state, (e) forming granules from the set solid state, and (f) selecting the granules based on size, preferably granules having an average diameter of 25 - 10,000 µm.
Unlike the processes in the art, the manufacturing method according to the invention does not necessitate a precipitation reaction of calcium phosphate and bioactive material from two calcium salt solutions onto a substrate granule.

In one aspect of the invention the weight ratio of the calcium phosphate matrix to the bioactive material in step (c) is between 10:0.0001 and 10:1, more preferably between 10: 0.0005 and 10:0.2, more preferably between 10:0.005 and 10:0.1, more preferably between 10:0.005 and 10:0.001, even more preferably between 10:0.01 and 10:0.0005.

In step (c) components (a) and (b) are preferably mixed to form a homogenous or even distribution of bioactive material dispersed in a matrix of calcium phosphate. This preferably means that the variation in concentration of the bioactive material is less than 40%, preferably less than 30%, more preferably less than 20%, even more preferably less than 10%, most preferably less than 5 % throughout set solid state after setting (d).

The term aqueous solution as applied in step (b) means a water-based liquid comprising at least 80 wt% water, preferably at least 85 wt%, more preferably at least 90 wt%, more preferably at least 95 wt%, based on the total weight of the liquid.

In one aspect of the invention the amount of bioactive material in the aqueous liquid (b) is preferably less than 1 wt%, more preferably less than 0.5 wt%, even more preferably less than 0.1 wt%, even more preferably less than 0.05 wt%, based on the total weight of the liquid. In a preferred embodiment, the amount of bioactive material is at least 0.005 wt%, preferably at least 0.01 wt%, preferably at least 0.02 wt% based on total weight of the liquid.

In one aspect of the invention the method to prepare the bioactive bone repair substrate is performed in a batchwise manner. In one aspect of the method a calcium phosphate powder is mixed with aqueous liquid comprising bioactive material at a powder to liquid ratio between 4:1 and 1.5:1, more preferably between 3:1 and 1.5:1, more preferably 2.5:1 and 1.5:1. In one aspect 200 grams of a calcium phosphate powder are mixed with 100 grams of aqueous liquid comprising bioactive material.

The method of preparing the bioactive bone repair substrate according to the invention is preferably carried out within a temperature range of 15 - 85°C, preferably 20 - 80 °C, preferably 30 - 80 °C, more preferably 30 - 60 °C, most preferably 30 - 35 °C. The method may preferably also be carried out at room temperature. The temperature can be used to influence the rate of setting. In one aspect of the invention the method of the invention does not involve pH for a parameter (i.e. is pH independent) and the method does not involve precipitation of the bioactive material and/or the calcium phosphate powder.

'Setting' refers to the reaction from water with calcium phosphate. The action of changing from a suspension to a solid state is called 'setting'. Setting of calcium phosphate is a traditional process. Typical setting times are in the range of 20 minutes to 3 days.

'Solid state' as used in the application means calcium phosphate that underwent hydration reaction in the presence of a liquid and thereby became solidified or set to a solid state.

In a further aspect the method according to the invention provides for the generation of bioactive bone repair substrate that is shelf-stable, i.e. they can be stored at room temperature for at least 6 months, preferably at least 1 year, more preferably at least up to 2 years. The shelf-stability is a consequence of the manufacturing method wherein the bioactive material is present throughout the granules and remains dry after setting.

### Granules

As used herein, osteoconductive materials refer to any material forming a matrix which facilitates the ingrowth or ongrowth of osteoblastic cells including osteoblasts, preosteoblasts, osteoprogenitor cells, mesenchymal stem cells and other cells which are capable of differentiating into or otherwise promoting the development of cells that synthesize and/or maintain bone tissue. In preferred embodiments of the present invention, the osteoconductive material is a granule comprising a calcium phosphate matrix to provide sustained release of a biological active material, preferably an osteoinductive substance that is loaded into and distributed through a calcium phosphate matrix.

As used herein, an "osteoinductive material" refers to material that is capable of inducing bone formation (i.e. a material having osteogenic properties) when implanted in a body and includes demineralized bone matrix and osteoinductive factors. The term osteoinductive factor refers for example to the materials described as bone growth factors. Demineralized bone matrix and bone morphogenic proteins (BMP's) are considered osteoinductive substances or osteoinductive materials that are able to enhance bone growth.

The granules used in the bone repair substrate of the invention are generally made of calcium phosphate(s) having a composition and architecture appropriate to remain in place and to release bioactive material over time intervals optimal for the formation and healing of bone. While these characteristics may vary between applications, exemplary compositions of the granules of bone repair substrate generally include granules comprising a calcium phosphates matrix such as monocalcium phosphate monohydrate, dicalcium phosphate, dicalcium phosphate dehydrate, octocalcium phosphate, precipitated hydroxyapatite, precipitated amorphous calcium phosphate, monocalcium phosphate, alpha-tricalcium phosphate (α-TCP), beta-tricalcium phosphate (β-TCP), sintered hydroxyapatite, oxyapatite, tetracalcium phosphate, hydroxyapatite, calcium-deficient hydroxyapatite, and combinations thereof. In one aspect the selection of calcium phosphates determines in part the releaseprofile of bioactive material from the bioactive bone repair substrate.

The granules used in the bone repair substrates according to the invention generally comprise interconnected micropores having a porosity of about 1 to 50%, more preferably between 5 and 45%, more preferably between 10 and 30%. The porosity is the percentage of void space formed by the interconnected micropores within the granules per volume unit of granules. Micropores are pores within the granules used in the bone repair substrate that are generally interconnected within the granules, having a diameter of less than about 10 micrometer. In a preferred embodiment the diameter of the micropores lies between 0.05 and 10 µm. The microporosity provided by the interconnected micropores in the granules provides the advantage that the granules have a larger surface area and are readily integrated into healing bone. Microporosity can be measured by Scanning Electron Microscopy (SEM), using ISO13175-3.

The granules used in the bone repair substrate according to the invention preferably do not have macropores within the granules. Macropores within the granules are defined as pores within a granule having a diameter of about 40 to 100 micrometer. The granules used in the bone repair substate according to the invention exhibit interconnected micropores but substantially no macropores within the granules. The granules used in the bone repair substrate are substantially free of macropores within the granules; less than 2% of the granules have macropores, more preferably less than 1% of the granules have macropores, most preferably less than 0.5% of the granules have macropores within the granules.

In one aspect the bone repair substrate has a macroporosity formed by the pores between the granules of the bone repair substrate of about 50%, more preferably of about 60%, more preferably of about 70%, even more preferably of about 80%. The macroporosity of the bone repair substrate does not result from macropores within the granules of the bone repair substrate. The bone repair substrate preferably has a macroporosity between 50% and 90%, more preferably between 60% and 80%, most preferably between 70% and 80%. The granules themselves as used in the bone repair substrate according to the invention do not have macropores. Macroporosity as formed by the pores between the granules of the bone repair substrate is assessed using SEM.

The bioactive bone repair substrate is in the form of granules. Diameters apply to the largest dimension of the granule unless stated otherwise. The granules preferably have an average diameter between 25 - 10,000 µm, preferably between 50 - 5,000 µm more preferably 100 - 2,500 µm, more preferably 125 - 2,000 µm, even more preferably 250 - 2,000 µm. The diameter can be determined using SEM or sieve analysis according to ISO13175-3.

The granules of the bone repair substrate are formed according to the described process, the size of the granules being obtained using generally known processes such as crushing, milling and the like, followed by size selection using techniques known in the art such as sieving. Where the desired particles are obtained by sieving, this may preferably be achieved by "dry" sieving. Reference is made to ISO standard number ISO13175-3 setting out such methods known in the art.

### Bioactive material

The bioactive bone repair substrate comprises granules formed by the method according to the invention wherein the bioactive material is homogeneously distributed throughout the set solid state, followed by forming granules of the solid state and selecting the appropriate granule size distribution.

The bioactive material distribution in the granules is based on the homogenous bioactive material concentration in the set solid state, and the sizing of the granules. In one aspect the bioactive bone repair substrate comprises granules obtained from the solid state wherein at least 10 vol% of the granules comprises bioactive material, preferably at least 15 vol%, more preferably at least 20 vol%, more preferably at least 30 vol%, more preferably at least 35 vol%, more preferably at least 40 vol%, even more preferably at least 50 vol%, most preferably at least 60 vol%, even more preferably at least 70 vol%, particularly at least 80 vol%, more particularly at least 90 vol%, even more particularly 100 vol% of the granules in the substrate comprise said bioactive material.

In one aspect of the invention the bioactive material used in the bioactive bone repair substrate forms at least 0.001 wt% relative to the total weight of the bioactive bone repair substrate, more preferably at least 0.005 wt%, more preferably at least 0.01 wt%, even more preferably 0.02 wt% relative to the total weight of the bioactive bone repair substrate. In a further aspect of the invention the bioactive material forms at most 0.2 wt% of the total weight of the bioactive bone repair substrate, more preferably at most 0.15 wt%, more preferably at most 0.1 wt%, more preferably at most 0.02 wt% relative to the total weight of the bioactive bone repair substrate.

Typically the bioactive material used in the bioactive bone repair substrate forms between 0.001 and 0.2 wt%, preferably between 0.002 and 0.1 wt%, more preferably between 0.005 and 0.05 wt%, more preferably between 0.010 and 0.03 wt% of the total weight of the bioactive bone repair granules.

In one aspect about 100% of the calcium phosphate granules comprise bioactive material. In a most preferred embodiment, the bioactive material is evenly or homogeneously distributed throughout the individual granules. This homogeneous distribution means that the variation in concentration of the bioactive material is preferably less than 40%, preferably less than 30%, more preferably less than 20%, even more preferably less than 10%, most preferably less than 5%. Additionally, it is preferred that the same maximum variation in average bioactive material concentrations applies between granules.

The distribution of bioactive material through the bone repair substrate and the associated sustained release properties can be characterized by a release test. A suitable release test to assess sustained release properties of bioactive material according to the invention is an ELISA test or a quantitative protein assay. 'Sustained release' as used herein means that the release of bioactive material lasts and can be measured and quantified using a release test preferably for at least 2 weeks, preferably at least 4 weeks, more preferably at least 6 weeks, more preferably at least 8 weeks, more preferably at least 10 weeks, even more preferably at least 12 weeks.

An ELISA test on a substrate immersed in *in vitro* conditions, preferably in a saline medium, is a suitable release test to assess release properties of the bioactive material. With the appropriate ELISA toolkit (e.g. BMP-2 ELISA (R&D Systems catalog #DBP200)) the release of the bioactive material from granules formed from the solid state and incubated in saline is assessed, allowing for a quantitative measurement of release of the levels of bioactive material from the bioactive bone repair granules into saline over time. In one aspect the sustained release of bioactive material is determined *in vitro* by incubating a set amount of bone repair substrate in a set volume of saline. The amount of bioactive material released in saline is followed by the quantification of the amount of bioactive material in supernatant aliquots taken at different points in time, such as but not limited to for example daily for 2 weeks, preferably for 4 weeks, more preferably for 6 weeks. Aliquots can be taken from the supernatant and the concentration of bioactive material in the saline can be measured via ELISA at longitudinal time points until no increase in the bioactive material concentration of the supernatant can be detected. The skilled person is aware of conventional ELISA assays and ways to work within the detection ranges of such ELISA assays. For example, ELISA kits for the quantitation of protein levels of human, rat, or mouse BMP-2 or human BMP-7 are commercially available, for example, from R&D Systems (catalog #DBP200, PDBP200, SBP200 or catalog #DY354 respectively). In one embodiment, 'sustained release' means that the release of bioactive material lasts preferably at least 2 weeks, preferably at least 4 weeks, more preferably at least 6 weeks, more preferably at least 8 weeks, more preferably at least 10 weeks, even more preferably at least 12 weeks after start of the *in vitro* test, and the test conditions are adapted to those time spans. The release kinetics of a selection of at least one bioactive factor such as BMP-2 is representative of the release of other bioactive factors from the bioactive bone repair substrate comprising calcium phosphate granules and bioactive material. Detection of the tested bioactive factor above the test's detection limit is generally considered as release.

The sustained release properties of the bioactive material can further be characterized by a quantitative protein assay such as a Bradford protein assay (BioRad #500-0201). Therefore substrate is immersed in *in vitro* conditions, preferably in a saline medium. Aliquots from the medium are sampled at different time points during immersion and protein levels in the supernatant are quantified. Aliquots are taken at different points in time, such as but not limited to for example daily for 2 weeks, preferably for 4 weeks, more preferably for 6 weeks. The protein levels over time are indicative for the release of bioactive material from the bioactive bone repair substrate.

In one aspect the measured *in vitro* retention of the bioactive material in the bioactive bone repair substrate is preferably at least 50%, preferably at least 60%, more preferably at least 80%, even more preferably at least 85% after 17 days of the initial amount of bioactive material in the bioactive bone repair substrate.

In one aspect of the invention the bioactive bone repair granules comprise an additional coating with bioactive material wherein the concentration of bioactive material is about 5% to about 20%, preferably about 10% to about 20% higher than in the bioactive bone repair granule. An additional bioactive material coating may be provided to allow for an initial burst release of bioactive material.

In one embodiment the bioactive materials for use in the bone repair substrate generally include human growth factors. In an embodiment the bioactive materials for use in the bone repair substrate are derived from allogeneic or xenogeneic bone, in particular from demineralized bone matrix. The bioactive materials for use in the bone repair substrate and derived from allogeneic or xenogeneic bone are obtainable through methods as described herein and in the art in accordance with US 5,236,456. In an embodiment the bioactive material for use in the bone repair substrate may also comprise a mixture of bone derived bioactive materials and recombinant bioactive materials.

In one embodiment the bioactive materials for use in the bone repair substrate generally include one or more growth factors that stimulate the generation of osteoblasts from populations of precursor cells. In some embodiments, the osteoinductive material is a member of the transforming growth factor beta (TGF-β) superfamily such as BMP-2, BMP-3, BMP-4, BMP-5, BMP-6, BMP-7, BMP-9 or a member of the platelet derived growth factor (PDGF) family such as PIGF, PDGF-A, PDGF-B, PDGF-C, PDGF-D, PGF or VEGF, and/or mixtures thereof.

Demineralized bone matrix as used herein, refers to any material obtainable by removing mineral material from bone tissue according to conventional tissue bank techniques as described in Reddi et al. "Biochemical sequences in the transformation of normal fibroblasts in adolescent rats", Proc. Nat. Acad. Sci, 69 pp1601 - 5 (1972), Zhang et al. "Effect(s) of the Demineralization Process on the Osteoinductivity of Demineralized Bone Matrix" Journal of Periodontology, Volume 68, Issue 11 (1997) and according to US 6,189,537 and according to US 7,811,608. In a preferred embodiment, the demineralized bone matrix described herein include preparations containing less than 8% calcium and preferably not less than approximately 2% calcium by weight.

In one aspect bioactive materials extracted from bone and the granules from a calcium phosphate based matrix for use in the bioactive bone repair substrate are obtainable according to the method comprising the steps of (a) providing a calcium phosphate powder, (b) providing an aqueous liquid comprising a bioactive material from demineralized bone matrix preferably human bioactive material; (c) admixing components (a) and (b), to obtain a mixture, and (d) allowing the mixture to set to a solid state, forming granules from the set solid state, and (f) selecting the granules based on size, preferably granules having an average diameter of 25 - 10000 µm.

The bioactive material from bone may be provided in any suitable manner known in the art. Demineralized bone matrix products may serve as a source for extraction of bioactive material, which are also available commercially, including for instance, from AlloSource (Denver, CO, USA), KeraLink (Baltimore, Maryland), and others. The bone source may be selected from cortical, cancellous, or corticocancellous bone. Further the bone may be of allogeneic or xenogeneic origin, in a preferred embodiment the bone is human bone. Use of bioactive material from bone in tissue repair compositions is described in the art such as US 4,394,370 and US 5,405,390 and 5,236,456.

The bioactive material extracted from bone is obtainable by a method with the following steps: 1) demineralizing bone tissue to provide a demineralized bone matrix from which at least part of the original calcium content of the tissue has been removed, 2) subjecting the demineralized bone matrix to an acid-promoted step resulting in cleavage of intra-molecular bonds in collagen molecules to provide a cleavage product which is acid soluble upon heating, and exposing said product simultaneously with or subsequent to the acid-promoted step to temperatures between about 15 °C and 85 °C, more preferably between about 30°C and about 60°C and most preferably between about 30°C and about 35°C to provide a liquid osteogenic composition, 3) optionally lyophilizating and converting the material to a powder by standard techniques known in the art, and 4) optionally resolubilizating, or reconstituting the powder thus optained in an aqueous liquid so that the composition comprising the bioactive material is in suitable form for mixing. Optionally the solid form of the bioactive material may also be obtained by placing the viscous liquid solution in a cellulose dialysis bag, with the resulting salts removed by dialysis or similar processes known in the art.

In a preferred aspect of the bone demineralization method the bone is demineralized in step 1) at room temperature, preferably for about 1 to about 72 hours, preferably about 1 to 24hrs, and more preferably for about 1 to about 12 hours. The acids that may be used in the demineralization method to obtain bioactive material from demineralized bone include inorganic acids such as hydrochloric acid, as well as organic acids such as ascorbic cid, formic acid, acetic acid, peracetic acid, citric acid and proprionic acid. The bone is preferably demineralized by hydrochloric acid. The concentration of the acid used in the demineralization method is preferably about 0.5N to about 1.0N.

The concentration of the acid used in the acid-promoted treatment of step 2) is preferably from about 2.0N to about 3.0N. The acid-promoted treatment of step 2) is preferably performed with an organic acid. Alternatively step 2 may be performed during the demineralization time of step 1) within the preferred time range of 1 to 72 hours of step 3).

Bioactive material derived from allogeneic or xenogeneic bone, in particular bioactive material derived from demineralized bone matrix comprises a selection or all of the following factors: non-collagenous proteins including phosphoproteins, osteocalcin, matrix Gla protein; osteoinductive growth factors including bone morphogenic proteins (BMP) such as BMP-2, BMP-4, BMP-6 and BMP-7; factors that contribute to bone growth such as fibroblast growth factor-2 (FGF-2), insulin-like growth factor-I and II (IGF-I and IGF-II), platelet derived growth factor (PDGF), and transforming growth factor beta-1 (TGF-beta 1) and further type I collagen and traces of calcium.

In yet another embodiment the bioactive material for use in the method to prepare the bioactive bone repair granules includes one or more cytostatic compounds that inhibit the growth of bone cancer cells. In some embodiments the cytostatic is selected from doxorubicin, cisplatin, carboplatin, etoposide, fosfamide, methotrexate or vincristine or combinations thereof.

In one aspect, the bioactive material of the invention may comprise one or more of any of the above growth factors. In a preferred embodiment, the bioactive material comprises at least a growth factor such as defined here above, in a most preferred embodiment the bioactive material comprises osteoinductive and osteoconductive factors derived from allogeneic or xenogeneic bone, in particular from demineralized bone matrix.

In one aspect of the invention the bioactive material is derived from xenogeneic donor bone wherein the xenogeneic bone is of mammalian origin, preferably bovine, porcine or reindeer origin.

In one aspect of the invention the bioactive material becomes evenly or homogeneously distributed to the calcium phosphate matrix during the mixing of the calcium phosphate and the bioactive material. The bioactive material remains biologically active for a sustained period of time. Without being bound by theory it is believed that the incorporation of the bioactive material throughout and within the calcium phosphate-based substrate provides for a dry environment in which bioactivity remains stable, thus enabling stability over prolonged periods of time.

In one aspect the release of bioactive material from the bone repair granules obtained according to the method of the invention may require the same osteoclastic resorption observed for the release of bone morphogenic protein (BMP) from bone. Bioactive bone repair granules according to the invention are in a preferred aspect resorbed in vivo allowing for release of the bioactive material over an extended period of time.

The bioactive bone repair granules may be used to treat or prevent bone loss, promote union or knitting of a fracture, and/or otherwise increase bone mass or treat a bone condition in the patient. A mammal suitable for the therapeutic methods according to the invention includes a primate, human, rodent, canine, feline, bovine, ovine, equine, swine, caprine, porcine, reindeer and the like. The ability of an agent or composition of the invention to treat bone fractures and defects may be determined by using assays well known to the art.

In one aspect of the invention the bioactive bone repair substrate comprising the granules obtainable by the method according to the invention are for therapeutic use in the treatment of bone defects in subjects in need thereof. In one aspect the bioactive bone repair substrate is for use as a medical device.

The products according to the invention generally have characteristics tailored to facilitate bone growth and healing over a sustained period of time. The release kinetics of the bioactive material is such that bone healing, growth and/or regeneration is improved. Additionally the structure of the granules according to the invention facilitates the formation of new bone without impairing the healing process, in a preferred embodiment the bone repair substrate is biodegradable. The bioactive bone repair granules are of particular use in mammals suffering from complex or large bone injury or suffering from impaired bone turnover processes such as those suffering from osteoporosis, diabetes mellitus and/or obesitas. It is believed that sustained release of bioactive material allows to induce and support bone formation. Without being bound by theory it is also believed that the sustained release of bioactive material from the bioactive bone repair substrate supports the physiological bone repair response without inducing unphysiological extra bone growth due to excess quantities of bioactive material being released.

The present invention has been described above with reference to a number of exemplary embodiments. Modifications and alternative implementations of some parts or elements are possible, and are included in the scope of protection as defined in the appended claims.

### Examples

The following examples illustrate the invention.

### Example 1 - synthesis of bioactive calcium deficient hydroxyapatite granules

An exemplary method to prepare the granules according to the invention comprises the mixing of components A and B:

### Component A: a powder

The powder consists of an equimolar mixture of tetracalcium phosphate (TTCP) and dicalciumphosphate (DCPD).

TTCP is synthesized from a solid state reaction between CaHPO₄ and CaCOs (J.T. Baker Chemical, Phillipsburg, NJ) at 1500°C for 6 h. The TTCP is then ground and sieved to obtain particles with sizes ranging from about 1-60 µm, with a median size of 20 µm.

Attempts using commercial DCPD resulted in long setting times; therefore, DCPD is synthesized. Briefly, DCPD is precipitated by increasing the pH of a DCPD-monocalcium phosphate monohydrate solution from 1.90 to 3.5 via CaCOs addition. The end pH is below the DCPD-hydroxyapatite singular point of 4.2 to prevent hydroxyapatite precipitation. The collected DCPD is washed and dried in air. The DCPD is subsequently ground to obtain a particle size range of 0.5-4 µm, with a median size of 1.3 µm. The DCPD powder is then mixed with TTCP at a molar ratio of 1:1 to form the component A powder for the calcium phosphate matrix.

### Component B: a liquid

The liquid comprised sodium phosphate as a hardening accelerator and hydroxypropyl methylcellulose (HPMC) as a gelling agent, following prior art.

A sodium phosphate solution (a mixture of Na₂HPO₄ and NaH₂PO₄ with a total ionic PO₄ concentration of 3 mol/L; Abbott, North Chicago, IL) are diluted with distilled water to obtain a sodium phosphate concentration of 0.2 mol/L. Then HPMC (Sigma-Aldrich, St. Louis, MO; viscosity = 100,000 centipoises at 2% by mass in water) is added to the solution at a HPMC mass fraction of 1% following a previous study. The bioactive material is added to the solution.

Component A and B are subsequently mixed in a powder (A) to liquid (B) ratio of 1:4 followed by settlement of the material under controlled conditions (25 °C, 75% humidity) for 3 days to set to a calcium phosphate solid state.

Once the solid state is reached the material is crushed using a jaw crusher. Subsequently the granules thus obtained are sieved to obtain granules in a desired size range between 250 - 2000 µm.

### Example 2 - Osteogenic (Bioactive) material from human bone

Human bone is defatted and disinfected by incubation for 1 hour in a 70% ethanol solution.

Subsequently bone is pulverized and sieved to an average particle size of about 500 µm. Following defatting and grinding, the bone is immersed in in 0.5N hydrochloric acid for 12 hours to effect demineralization of the bone particles. After the demineralization operation has been completed, the bone tissue is then subjected to a separate acid-promoted treatment accompanied with heating involving incubation with 2N hydrochloric acid for 2 hours at a temperature of about 40 °C. The bone particles undergo a phase change induced by this acid-promoted treatment and turn into a pulp. Once the bone material has become a pulp the excess acid is removed by dializing against deionized water until the pH is about 3. The bone extract is washed and centrifuged at 3000g for 15 minutes three times in deionized water. The pellet is then lyophilized to yield a dry powder. 10 grams of lyophilized powder is reconstituted in 20 ml of water and mixed into liquid component B from Example 1.

### Example 3 - Measurement of Release kinetics of BMP-2 from bioactive bone repair granules

The release of bioactive material from the bioactive bone repair granules is determined by incubating 10 mg of granules in triplicate for each time point at 37 °C in saline. As a negative control granules without bioactive material incorporated therein are incubated. Granules are incubated for 10 weeks under sterile circumstances. In the first week every day a sample of 100 µl of supernatant is taken, followed by sampling twice a week in weeks 2 till 10. Supernatants are stored at -80°C until analysis. The amounts of BMP-2 in the supernatant is assessed by a BMP-2 ELISA (R&D Systems catalog #DBP200) and cumulative BMP-2 release over time is calculated. Further a Bradford protein assay (BioRad #500-0201) is performed to measure the concentration of protein released into the supernatant over time.

### Example 4

### Materials:

- Alpha Tri-Calcium Phosphate (particle size <10 micron) = Component A
- Deionized water (ASTM D1193-06)
- Sodium dihydrogen phosphate dihydrate (NaH₂PO₄ * 2 H₂O, CAS nr: 13472-35-0)
- Bioactive material prepared according to example 2

Prepare component B by dissolving 4 grams of Sodium dihydrogen phosphate dihydrate in 96 ml of deionized water. Add 0.02 grams of Bioactive material as made according to example 2 to the solution.

Mix component A (the powder) and component B (the liquid) in a ratio of 2:1 followed by settlement of the material under controlled conditions (25 °C, 75% humidity) for 3 days to set to a calcium phosphate solid state.

Once the solid state is reached the material is crushed using a jaw crusher. Subsequently the granules thus obtained are sieved to obtain granules in a desired size range between 250 - 2000µm.

### Example 5

### Materials:

### Setting liquid (component B):

- 4 grams of sodium phosphate [Sodium dihydrogen phosphate dihydrate, Merck 1.06342-0250; K95536642820]
- 96 ml of deionized water.

The setting liquid was prepared by dissolving 4 grams of Sodium dihydrogen phosphate dihydrate in 96 ml of deionized water.

### Granules:

- 30 grams of alpha-Tri-Calcium Phosphate (component A) [SAMRM041218A]
- 31 mg Bovine Serum Albumin (BSA) [Sigma lot 072K1179]
- 15 ml Sodium Phosphate solution.

Thirty grams of alpha-TCP and 31 milligrams of BSA were added to 15 ml sodium phosphate solution and mixed thoroughly. The mixture was allowed to set for one day at 37 °C to a set solid state. The set solid state was grind with mortar and pestle into granules.

### MES buffer:

- 9.76 grams MES [Sigma M8250 Lot#SLBS6258]
- 75 ml deionized water

The MES buffer was prepared by dissolving 9.76g of MES in 75 ml deionized water, followed by setting the pH to 5.5 with NAOH (10N) and filtering of the resulting solution. Subsequently water was added till 100 ml and the solution was filtered again.

One gram of the resulting BSA-loaded calcium deficient hydroxyapatite granules was incubated in 5 ml of MES buffer at 37°C. Supernatant samples were obtained at 4 days and 17 days of incubation. The concentration of BSA released from the granules was assessed using a quantitative protein assay showing a gradual release of BSA from the granules [figure 1]. As illustrated in the graph more than 90% of the incorporated BSA remained bound to the granules over a period of 17 days.

## Claims

1. A method for preparing bioactive bone repair substrate comprising granules from a calcium phosphate based bone repair matrix and at least one bioactive material dispersed therein, said method comprising: (a) providing a calcium phosphate powder, (b) providing an aqueous solution comprising a bioactive material derived from bone; (c) admixing a calcium phosphate powder (a) and an aqueous solution comprising a bioactive material derived from bone (b), to obtain a mixture, (d) allowing the mixture to set to a solid state, (e) forming granules from the set solid state, and (f) selecting the granules based on size, preferably granules having an average diameter between 25 and 10,000 µm.

2. The method according to claim 1, wherein the method comprises obtaining bioactive material for method step b) according to the following steps; 1) demineralizing bone tissue to provide a demineralized bone matrix from which at least part of the original calcium content of the tissue has been removed 2) subjecting the demineralized bone matrix to an acid-promoted step and exposing the demineralized bone matrix material simultaneously with, or subsequent to the acid-promoted step to temperatures between 15°C and 85°C, more preferably between 30°C and 60°C and most preferably between 30°C and 35°C, 3) optionally lyophilizating and converting the material to a powder, and 4) optionally resolubilizating the powder thus obtained in an aqueous liquid.

3. The method according to claims 1 or 2 wherein the bioactive material is homogeneously distributed through (d) the set solid state mixture.

4. The method according to any of claims 1 - 3 wherein the calcium phosphate powder is selected from the group consisting of monocalcium phosphate monohydrate, dicalcium phosphate, dicalcium phosphate dehydrate, octocalcium phosphate, precipitated hydroxyapatite, precipitated amorphous calcium phosphate, monocalcium phosphate, alpha-tricalcium phosphate (α-TCP), beta-tricalcium phosphate (β-TCP), sintered hydroxyapatite, oxyapatite, tetracalcium phosphate, hydroxyapatite, calcium-deficient hydroxyapatite, and combinations thereof.

5. The method according to any of claims 1 - 4 wherein granules are formed and selected in a size range of 50 - 5000 µm.

6. The bioactive bone repair substrate obtainable by the method of claims 1 - 5, for therapeutic use in the treatment of bone defects in subjects in need thereof.

7. Bioactive bone repair substrate obtainable according to the method of claims 1 - 5.

## Patentansprüche

1. Verfahren zum Herstellen eines bioaktiven Knochenreparatursubstrats, das Granulat aus einer auf Calciumphosphat basierenden Knochenreparaturmatrix und mindestens ein darin dispergiertes bioaktives Material umfasst, wobei das besagte Verfahren Folgendes umfasst: (a) Bereitstellen eines Calciumphosphatpulvers, (b) Bereitstellen einer wässrigen Lösung, die ein aus Knochen gewonnenes bioaktives Material umfasst; (c) Vermischen eines Calciumphosphatpulvers (a) und einer wässrigen Lösung, die ein aus Knochen (b) gewonnenes bioaktives Material umfasst, um eine Mischung zu erhalten, (d) Erstarrenlassen der Mischung in einen festen Zustand, (e) Bilden von Granulat aus dem erstarrten festen Zustand und (f) Auswählen des Granulats basierend auf der Größe, vorzugsweise Granulat mit einem durchschnittlichen Durchmesser zwischen 25 und 10.000 µm.

2. Verfahren nach Anspruch 1, wobei das Verfahren das Erhalten eines bioaktiven Materials für Verfahrensschritt b) gemäß den folgenden Schritten umfasst: 1) Demineralisieren von Knochengewebe, um eine demineralisierte Knochenmatrix bereitzustellen, aus der mindestens ein Teil des ursprünglichen Calciumgehalts des Gewebes entfernt wurde, 2) Unterziehen der demineralisierten Knochenmatrix einem säuregeförderten Schritt und Aussetzen des demineralisierten Knochenmatrixmaterials gleichzeitig mit oder anschließend an den säuregeförderten Schritt Temperaturen zwischen 15 °C und 85 °C, besonders bevorzugt zwischen 30 °C und 60 °C und am bevorzugtesten zwischen 30 °C und 35 °C, 3) gegebenenfalls Lyophilisieren und Umwandeln des Materials in ein Pulver und 4) gegebenenfalls Resolubilisieren des so erhaltenen Pulvers in einer wässrigen Flüssigkeit.

3. Verfahren nach Anspruch 1 oder 2, wobei das bioaktive Material homogen durch (d) die Mischung des erstarrten festen Zustands verteilt ist.

4. Verfahren nach einem der Ansprüche 1 - 3, wobei das Calciumphosphatpulver aus der Gruppe ausgewählt ist, die aus Folgendem besteht: Monocalciumphosphat-Monohydrat, Dicalciumphosphat, Dicalciumphosphat-Dehydrat, Octocalciumphosphat, ausgefälltem Hydroxyapatit, ausgefälltem amorphem Calciumphosphat, Monocalciumphosphat, Alpha-Tricalciumphosphat (α-TCP), Beta-Tricalciumphosphat (β-TCP), gesintertem Hydroxyapatit, Oxyapatit, Tetracalciumphosphat, Hydroxyapatit, calciumarmem Hydroxyapatit und Kombinationen davon.

5. Verfahren nach einem der Ansprüche 1 - 4, wobei Granulat in einem Größenbereich von 50 - 5000 µm gebildet und ausgewählt wird.

6. Bioaktives Knochenreparatursubstrat, erhältlich durch das Verfahren der Ansprüche 1 - 5, zur therapeutischen Verwendung bei der Behandlung von Knochendefekten bei Patienten, die dies benötigen.

7. Bioaktives Knochenreparatursubstrat, erhältlich nach dem Verfahren der Ansprüche 1 - 5.

## Revendications

1. Procédé de préparation d'un substrat bioactif de réparation osseuse comprenant des granules d'une matrice de réparation osseuse à base de phosphate de calcium et au moins un matériau bioactif dispersé à l'intérieur, ledit procédé comprenant : (a) fournir une poudre de phosphate de calcium, (b) fournir une solution aqueuse comprenant un matériau bioactif dérivé d'os ; (c) mélanger une poudre de phosphate de calcium (a) et une solution aqueuse comprenant un matériau bioactif dérivé d'os (b), pour obtenir un mélange, (d) permettre au mélange de prendre un état solide, (e) former des granules à partir de l'état solide fixé, et (f) sélectionner les granules en fonction de leur taille, de préférence des granules ayant un diamètre moyen entre 25 et 10000 µm.

2. Procédé selon la revendication 1, où le procédé comprend l'obtention d'un matériau bioactif pour l'étape b) du procédé selon les étapes suivantes ; 1) déminéraliser le tissu osseux pour fournir une matrice osseuse déminéralisée de laquelle au moins une partie de la teneur originale en calcium du tissu a été éliminée, 2) soumettre la matrice osseuse déminéralisée à une étape favorisée par acide et exposer le matériau de la matrice osseuse déminéralisée simultanément avec, ou après l'étape favorisée par acide à des températures entre 15°C et 85°C, plus préférablement entre 30°C et 60°C et le plus préférablement entre 30°C et 35°C, 3) facultativement lyophiliser et convertir le matériau en une poudre, et 4) facultativement resolubiliser la poudre ainsi obtenue dans un liquide aqueux.

3. Procédé selon les revendications 1 ou 2, où le matériau bioactif est distribué de manière homogène à travers (d) le mélange à l'état solide fixé.

4. Procédé selon l'une quelconque des revendications 1 à 3, où la poudre de phosphate de calcium est sélectionnée dans le groupe consistant en phosphate monocalcique monohydraté, phosphate dicalcique, phosphate dicalcique déshydraté, phosphate octocalcique, hydroxyapatite précipitée, phosphate de calcium amorphe précipité, phosphate monocalcique, phosphate alpha-tricalcique (α-TCP), phosphate bêta-tricalcique (β-TCP), hydroxyapatite frittée, oxyapatite, phosphate tétracalcique, hydroxyapatite, hydroxyapatite déficiente en calcium, et des combinaisons de ceux-ci.

5. Procédé selon l'une quelconque des revendications 1 à 4, où des granules sont formés et sélectionnés dans une gamme de taille de 50 à 5 000 µm.

6. Substrat bioactif de réparation osseuse pouvant être obtenu par le procédé des revendications 1 à 5, pour une utilisation thérapeutique dans le traitement de défauts osseux chez des sujets en ayant besoin.

7. Substrat bioactif de réparation osseuse pouvant être obtenu selon le procédé des revendications 1 à 5.
